(19) Europäisches Patentamt
European Patent Office
Office européen des brevets

(11) Veröffentlichungsnummer: **0 420 804 A2**

(12) **EUROPÄISCHE PATENTANMELDUNG**

(21) Anmeldenummer: **90810710.5**

(22) Anmeldetag: **18.09.90**

(51) Int. Cl.5: **C07D 213/643**, C07D 213/85, C07D 239/34, C07D 239/38, A01N 43/40, A01N 43/54

(30) Priorität: **26.09.89 CH 3481/89**

(43) Veröffentlichungstag der Anmeldung: **03.04.91 Patentblatt 91/14**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Maienfisch, Peter, Dr.**
**Traugott Meyer-Strasse 5**
**CH-4147 Aesch(CH)**
Erfinder: **Hildenbrand, Christof, Dr.**
**Birsigstrasse 80**
**CH-4045 Basel(CH)**
Erfinder: **Gehret, Jean-Claude, Dr.**
**Im Aeschfeld 12**
**CH-4147 Aesch(CH)**

(54) **Anthelmintika.**

(57) Es wurden anthelmintisch aktive Verbindungen der Formel I beschrieben

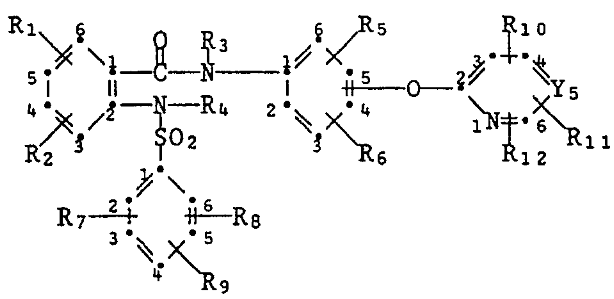

worin
$R_1$ für Wasserstoff, Halogen, $C_1$-$C_2$-Alkyl, $C_1$-$C_2$-Thioalkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht;
$R_2$ für Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy steht;
$R_3$ Wasserstoff oder $C_1$-$C_2$-Alkyl bedeutet;
$R_4$ Wasserstoff oder $C_1$-$C_2$-Alkyl bedeutet;
$R_5$ für Wasserstoff, Halogen oder $C_1$-$C_5$-Alkyl steht;
$R_6$ für Wasserstoff, Halogen oder $C_1$-$C_5$-Alkyl steht;
$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;
$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;
$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;
$R_{12}$ für Wasserstoff oder Halogen steht; und
Y =CH- oder =N- repräsentiert; unter Einschluss ihrer physiologisch verträglichen Säureadditionssalze, sowie

EP 0 420 804 A2

deren Herstellung und Verwendung und neue Zwischenprodukte.

## ANTHELMINTIKA

Die vorliegende Erfindung betrifft neue substituierte Anthranilsäure-Derivate mit anthelmintischer Wirksamkeit, anthelmintische Mittel auf der Basis dieser neuen Wirksubstanzen, die Verwendung der Aktivsubstanzen und Mittel zur Bekämpfung von Helminthen, insbesondere Nematoden, Cestoden und Trematoden bei Warmblütern, insbesondere bei Säugetieren und vorzugsweise bei Haus- und Nutztieren. Die Erfindung betrifft gleichermassen die Herstellung der Wirkstoffe und der Mittel und neue Zwischenprodukte für die Herstellung der Aktivsubstanzen.

Die neuen Anthranilsäure-Derivate haben die nachstehende Formel I

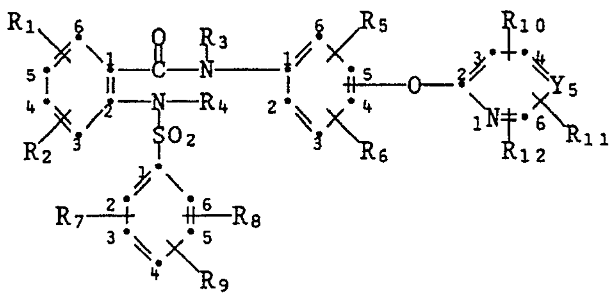

(I)

worin

$R_1$ für Wasserstoff, Halogen, $C_1$-$C_2$-Alkyl, $C_1$-$C_2$-Thioalkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht;
$R_2$ für Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy steht;
$R_3$ Wasserstoff oder $C_1$-$C_2$-Alkyl bedeutet;
$R_4$ Wasserstoff oder $C_1$-$C_2$-Alkyl bedeutet;
$R_5$ für Wasserstoff, Halogen oder $C_1$-$C_5$-Alkyl steht;
$R_6$ für Wasserstoff, Halogen oder $C_1$-$C_5$-Alkyl steht;
$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;
$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;
$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;
$R_{12}$ für Wasserstoff oder Halogen steht; und
Y =CH- oder =N- repräsentiert; unter Einschluss ihrer physiologisch verträglichen Säureadditionssalze.

Erwähnenswert ist folgende Untergruppe von Verbindungen der Formel I worin
$R_1$ für Wasserstoff, Halogen, $C_1$-$C_2$-Alkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht;
$R_2$ für Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy steht;
$R_3$ Wasserstoff oder $C_1$-$C_2$-Alkyl bedeutet;
$R_4$ Wasserstoff oder $C_1$-$C_2$-Alkyl bedeutet;
$R_5$ für Wasserstoff, Halogen oder $C_1$-$C_5$-Alkyl steht;
$R_6$ für Wasserstoff, Halogen oder $C_1$-$C_5$-Alkyl steht;
$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;
$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;
$R_{11}$ für Wasserstoff, Halogen, $C_3$-$C_6$-Alkyl, $C_1$-$C_2$-Haloalkyl, $C_1$-$C_2$-Alkylthio, oder $C_3$-$C_6$-Cycloalkyl steht;
$R_{12}$ für Wasserstoff oder Halogen steht; und
Y =CH- oder =N- repräsentiert; unter Einschluss ihrer physiologisch verträglichen Säureadditionssalze.

Unter dem Begriff Alkyl selbst oder als Bestandteil eines anderen Substituenten sind im Rahmen vorliegender Erfindung je nach Anzahl der angegebenen Kohlenstoffatome folgende geradkettige und

verzweigte Gruppen zu verstehen, z.B.: Methyl, Ethyl, n-Propyl, iso-Propyl, n-Butyl, sek.-Butyl, tert.Butyl, Isobutyl, etc.. Haloalkyl selbst oder als Bestandteile für Haloalkoxy steht für einen einfach bis perhalogenierten Alkylsubstituenten, wie z.B. für $CH_2Cl$, $CHCl_2$, $CCl_3$, $CH_2F$, $CHF_2$, $CF_3$, $CH_2Br$, $CHBr_2$, $CBr_3$, $CH_2J$, $CJ_3$, $CHClF$, $CHBrCl$, $CFBrCl$, $C_2F_5$, $CH_2CH_2Cl$, $CHClCH_3$, $C_2Cl_5$, $CHFCHCl_2$, etc., vorzugsweise für $CF_3$. Unter Halogen soll hier und im folgenden Fluor, Chlor, Brom oder Jod, vorzugsweise Fluor, Chlor oder Brom, vor allem jedoch Chlor verstanden werden.

Eine bevorzugte Untergruppe von Verbindungen der Formel I bilden Substanzen, worin
$R_1$, $R_2$, $R_3$, $R_4$, $R_7$, $R_8$, $R_9$, $R_{12}$ und Y die unter Formel I angegebenen Bedeutungen haben;
$R_5$ für Wasserstoff, Halogen oder $C_1$-$C_3$-Alkyl steht;
$R_6$ für Wasserstoff, Halogen oder $C_1$-$C_3$-Alkyl steht;
$R_{10}$ für Wasserstoff, Halogen, $C_1$-$C_4$-Alkyl, $C_1$-$C_2$-Haloalkyl, $C_1$-$C_2$-Alkylthio, Cyclopropyl oder Cyano steht; und
$R_{11}$ für Wasserstoff, Halogen, $C_1$-$C_4$-Alkyl, $C_1$-$C_2$-Haloalkyl, $C_1$-$C_2$-Alkylthio oder Cyclopropyl steht; unter Einschluss ihrer physiologisch verträglichen Säureadditionssalze.

Eine weitere bevorzugte Gruppe bilden Verbindungen der Formel I, worin
$R_1$ für Wasserstoff, Halogen, Methyl, Methoxy oder Thiomethyl steht;
$R_2$ Wasserstoff, Methyl oder Halogen bedeutet;
$R_3$ Wasserstoff, Methyl oder Ethyl bedeutet;
$R_4$ Wasserstoff , Methyl oder Ethyl bedeutet;
$R_5$ für Wasserstoff, Halogen oder $C_1$-$C_4$-Alkyl steht;
$R_6$ für Wasserstoff, Halogen oder $C_1$-$C_4$-Alkyl steht;
$R_7$ Wasserstoff, Halogen, Methyl, Methoxy, $CF_3$ oder Nitro bedeutet;
$R_8$ für Wasserstoff oder Halogen steht;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ und $R_{11}$ unabhängig voneinander für Wasserstoff, Halogen, $C_1$-$C_4$-Alkyl, Methylthio, Trifluormethyl, $CF_2CCl_2F$, Cyclopropyl oder Cyano stehen;
$R_{12}$ für Wasserstoff oder Halogen steht; und
Y für =CH- oder =N- steht, wobei der Pyridinoxy- oder Pyrimidinoxy-Substituent über die 3- oder 4-Position des Phenylrings gebunden ist.

Andere Gruppen bevorzugter Verbindungen der Formel I bilden jene, worin $R_1$ für 5-Halogen (bevorzugt Chlor) oder 4-Halogen (bevorzugt Chlor) steht, $R_2$ Wasserstoff bedeutet und die übrigen Substituenten die unter Formel I angegebenen Bedeutungen haben, bzw. wie in den oben als bevorzugt genannten Gruppen definiert sind.

Im Rahmen der Formel I und den genannten Untergruppen sind diejenigen Vertreter bevorzugt bei denen sich der Substituent $R_1$ in 5-Position der Phenylgruppe befindet.

Zu den bevorzugten Einzelvertreter von Verbindungen der Formel I gehören:
5-Chlor-2-[(4-chlor-phenyl-sulfonyl)-amino]-benzoesäure-[4-(4-trifluormethyl-6-cyclopropyl-pyrimidin-2-yl-oxy)-anilid];
5-Chlor-2-[(4-chlor-phenyl-sulfonyl)-amino]-benzoesäure-[4-(3-chlor-5-trifluormethyl-pyridin-2-yl-oxy)-anilid];
5-Chlor-2-[(4-chlor-phenyl-sulfonyl)-amino]-benzoesäure-[4-(4-trifluormethyl-pyridin-2-yl-oxy)-anilid];
5-Chlor-2-[(4-chlor-phenyl-sulfonyl)-amino]-benzoesäure-[4-(4-trifluormethyl-6-tert.butyl-pyrimidin-2-yl-oxy)-anilid];
3,5-Dichlor-2-[(4-chlor-phenyl-sulfonyl)-amino]-benzoesäure-[4-(4-trifluormethyl-6-cyclopropyl-pyrimidin-2-yl-oxy)-anilid];
3,5-Dichlor-2-[(4-chlor-phenyl-sulfonyl)-amino]-benzoesäure-[4-(5-trifluormethyl-pyridin-2-yl-oxy)-anilid].

Dazu gehört auch: 4-Chlor-2-[(4-chlor-phenyl-sulfonyl)-amino]-benzoesäure-[4-(3-chlor-5-trifluormethyl-pyridin-2-yl-oxy)-anilid].

Es wurde nun gefunden, dass die erfindungsgemässen neuen Verbindungen der Formel I überraschenderweise ein sehr günstiges Wirkungsspektrum gegen tierparasitäre Helminthen besitzen, vor allem gegen solche, welche in Warmblütern, insbesondere Säugetieren, parasitieren. Die Verbindungen der Formel I sind gegen Nematoden sowie Cestoden und Trematoden mit gutem Erfolg anwendbar. Dabei zeichnen sie sich vor allem dadurch aus, dass sie auch gegen benzimidazol-resistente, insbesondere gegen thiabendazol-resistente Helminthen voll wirksam sind ("thiabendazol" bezeichnet den Wirkstoff 2-(Thiazol-4-yl)-benzimidazol).

Die Verbindungen der Formel I werden dadurch hergestellt, dass man
(A) eine Verbindung der Formel II

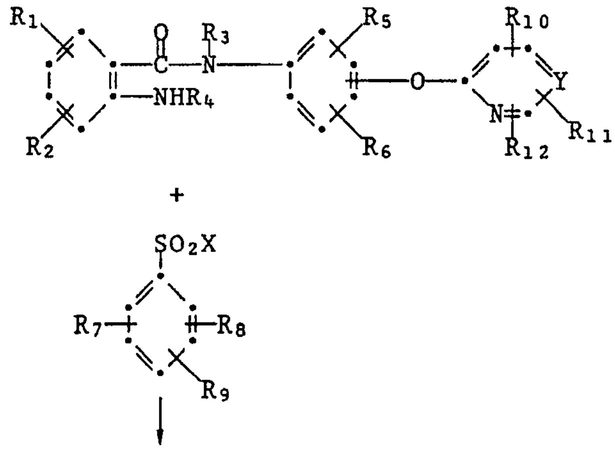

(II)

+

(III)

(I)

mit einem Benzolsulfonsäurehalogenid der Formel III, oder

(B) eine Verbindung der Formel IV

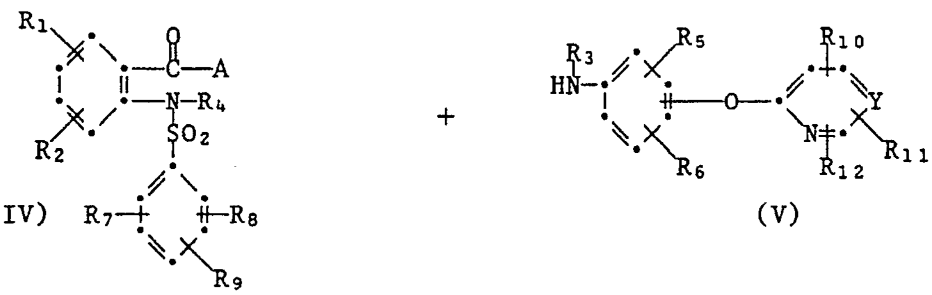

(IV) + (V) → (I)

mit einer Verbindung der Formel V umsetzt, wobei die Substituenten $R_1$ bis $R_{12}$ sowie Y in den Formeln II, III, IV und V die unter Formel I angegebenen Bedeutungen haben, X in Formel III für Halogen, vorzugsweise Chlor oder Brom, insbesondere Chlor steht und A in Formel IV für Halogen (bevorzugt Chlor oder Brom), OH oder eine zur Reaktion mit einer Amino gruppe befähigte Abgangsgruppe darstellt und gegebenenfalls eine Verbindung der Formel II worin $R_3$ und $R_4$ oder $R_3$ oder $R_4$ für Wasserstoff stehen, nachalkyliert.

Bei den Umsetzungen von (II) mit (III) sowie von (IV) mit (V) können als Basen z.B. tertiäre Amine (wie Triethylamin, Trimethylamin, Tripropylamin, N-Methylpiperidin, 1,4-Diazabicyclo(2,2,2)octan usw.), Pyridin und Pyridinbasen (wie 4-Dimethylaminopyridin, 4-Pyrrolidylaminopyridin, Picoline, Lutidin usw.) sowie Erdalkali- oder vorzugsweise Alkalialkoholate von Niederalkanolen (wie z.B. Natrium- oder Kaliumalkoholate des Methanols, Propanols, Ethanols, n-Butanols, iso-Butanols, tert.-Butanols usw.) eingesetzt werden. Bevorzugt ist Pyridin.

Als Lösungs- oder Verdünnungsmittel kommen beispielsweise folgende aprotische Solventien in Frage: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; z.T. N,N-alkylierte Amide wie Dimethylformamid; Dimethylsulfoxid; Ketone wie Aceton, Diethylketon, Methylethylketon und Gemische solcher Lösungsmittel untereinander. In manchen Fällen kann die Base selbst als Lösungsmittel dienen.

Die Reaktionstemperaturen liegen üblicherweise von -20° bis +150° C, vorzugsweise 0° bis +100° C. In Fällen, in denen A in Formel IV für OH steht, kann ein Kondensationsmittel wie Dicyclohexylcarbodiimid zugesetzt werden.

Das beschriebene Herstellungsverfahren ist, einschliesslich aller Teilschritte, ein wichtiger Bestandteil der vorliegenden Erfindung.

Die Verbindungen der Formel II sind neu und stellen einen Bestandteil der vorliegenden Erfindung dar, da in ihnen die Hauptcharakteristika der Endprodukte bereits vorgegeben sind.

Die Verbindungen der Formel II sind nach mehreren Verfahren herstellbar, so z.B. aus einer Verbindung der Formel VI

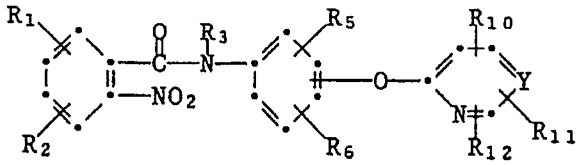

(VI)

(i) durch Hydrierung in Anwesenheit eines geeigneten Hydrierkatalysators und gegebenenfalls durch nachträgliche N-Alkylierung zur Einführung des Substituenten $R_4$, oder

(ii) durch chemische Reduktion, oder

(iii) durch Umsetzung einer Verbindung der Formel VII

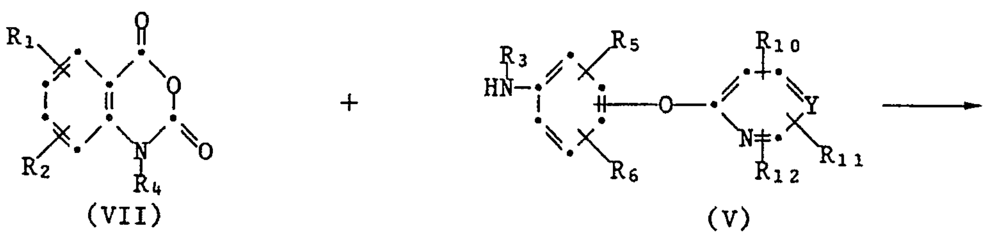

(VII) + (V) (II)

mit einer Verbindung der Formel V, wobei die Substituenten $R_1$, $R_2$, $R_3$, $R_4$, $R_5$, $R_6$, $R_{10}$, $R_{11}$ und $R_{12}$ sowie Y die unter Formel I angegebenen Bedeutungen haben.

Die Reaktionen (i) und (ii) können unter Normaldruck stattfinden und werden bevorzugt in Gegenwart eines inerten organischen Lösungs- oder Verdünnungsmittels durchgeführt, vorzugsweise in Gegenwart eines der weiter oben genannten Ether oder etherartigen Verbindungen, eines Esters wie Ethylacetat, Propylacetat oder Butylacetat oder eines Alkohols, insbesondere eines Alkanols wie Methanol, Ethanol, Propanol, etc.. Die Reaktionen werden im allgemeinen bei Temperaturen von 0° bis 80° C, vorzugsweise 10° bis 50° C, durchgeführt.

Als Katalysator in Verfahren (i) kommt beispielsweise Rh/C oder Raney-Nickel in Frage.

Die chemische Reduktion (ii) lässt sich beispielsweise mit Sn-(II)-chlorid/HCl durchführen.

Die Reaktion (iii) wird vorzugsweise in Gegenwart eines polaren, inerten organischen Lösungsmittels wie N,N-Dimethylformamid, N,N-Dimethylacetamid, Dimethylsulfoxid, Dioxan, Butanon, THF oder Dimethoxyethan durchgeführt. Die Reaktionstemperaturen liegen bei -10° bis +150° C, vorzugsweise 0° bis 100° C.

Die Nitroverbindungen der Formel VI sind ebenfalls neu und stellen einen Gegenstand der vorliegenden Erfindung dar.

Verbindungen der Formel VI können durch Umsetzung von Verbindungen der Formel VIII

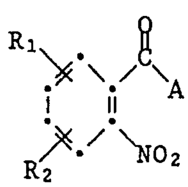

(VIII) + (V) (VI)

mit Verbindungen der Formel (V) hergestellt werden wobei A in Formel VIII OH oder eine reaktive Abgangsgruppe wie Halogen (bevorzugt Chlor oder Brom) oder einen aktivierten Esterrest wie z.B. Alkoxycarbonyloxy bedeutet. Diese Umsetzungen werden vorzugsweise in Gegenwart eines inerten, organischen Lösungsmittels wie Benzol, Toluol, $CH_2Cl_2$,Ether, DMF, DMSO, THF, Acetonitril oder Aceton durchgeführt, wobei vorteilhafterweise in Gegenwart einer Base wie Triethylamin, Pyridin, Picolin zwischen -20° C bis 150° C, vorzugsweise zwischen 0° C und 50° C gearbeitet wird. Wenn A = OH bedeutet, kann die Umsetzung auch in Gegenwart eines Kondensationsmittels wie z.B. Dicyclohexylcarbodiimid durchgeführt werden.

Verbindungen der Formel V sind bekannt oder können hergestellt werden z.B. durch Umsetzung einer Verbindung der Formel IX

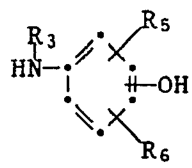

(IX)

mit einer Verbindung der Formel X

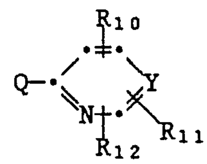

(X)

worin $R_3$, $R_5$, $R_6$, $R_{10}$, $R_{11}$ und $R_{12}$ sowie Y die unter Formel I angegebenen Bedeutungen haben und Q für eine übliche Abgangsgruppe steht. Die Reaktionstemperaturen liegen üblicherweise bei 0 °C bis 200 °C, vorzugsweise 10 °C bis 150 °C, wobei man in polaren inerten Lösungsmitteln wie z.B. N,N-Dimethylformamid, N,N-Dimethylacetamid vorzugsweise Dimethylsulfoxid arbeitet, unter Zusatz einer anorganischen Base wie Natriumcarbonat, Kaliumcarbonat, bevorzugt Kaliumhydroxid. Das bei diesen Umsetzungen entstehende Reaktionswasser kann mittels eines Schleppmittels wie z.B. $CH_2Cl_2$, Toluol, Benzol aus dem Reaktionsgemisch entfernt werden.

Q steht in Formel X entweder für eine der üblichen Abgangsgruppen, z.B. Halogen, insbesondere Chlor, Brom oder Jod; für eine Sulfonyloxygruppe, insbesondere Benzolsulfonyloxy, Paratosyloxy oder Niederalkylsulfonyloxy, bevorzugt Mesyloxy.

Verbindungen der Formel VII gewinnt man vorzugsweise durch Oxidation von Verbindungen der Formel XI

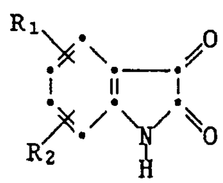

(XI)

Diese Herstellungsverfahren sind bekannt unter anderem aus DE 29 25 175 und Angewandte Chemie 92 , 196 (1980). Die aus diesen Verfahren gewonnenen Verbindungen der Formel VII ($R_4$ = H) lassen sich gegebenenfalls am Stickstoff alkylieren, zum Beispiel nach J. Heterocycl. Chem. 565 (1975).

Die Verbindungen der Formel XI und deren Herstellungsverfahren sind bekannt und können nach einer dem Fachmann geläufigen Methode hergestellt werden, vgl. Houben/Weyl Bd. 7/4, S. 5 ff.

Verbindungen der Formel IV (A = OH) können durch Verseifung von Verbindungen der Formel XII oder XIII

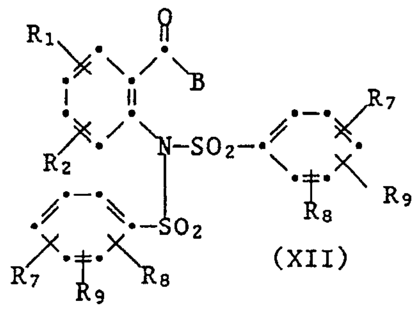

(XII)

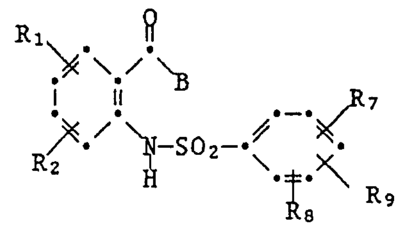

(XIII)

erhalten werden, wobei B für $OCH_3$ oder $OC_2H_5$ steht. Die erwähnte Verseifung geschieht vorzugsweise mit einer anorganischen Base wie NaOH oder KOH in Gegenwart eines Lösungsmittels wie Wasser und/oder eines Alkohols, vorzugsweise Methanol oder Ethanol, vgl. Houben/Weyl, 9, S. 609 ff.

Verbindungen der Formel IV (A = reaktive Abgangsgruppe) können nach dem Fachmann geläufigen Methoden z.B. aus Verbindungen der Formel IV (A = OH) hergestellt werden.

Die Verbindungen der Formel XII sind neu und stellen ebenfalls einen Gegenstand der vorliegenden Erfindung dar.

Die Verbindungen der Formeln XII und XIII sind zugänglich durch Umsetzung von Verbindungen der Formeln XIV, wobei B, $R_1$ und $R_2$ die oben angegebene Bedeutung haben,

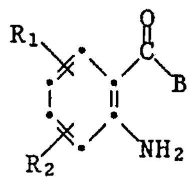

(XIV)

mit Verbindungen der Formel III, wobei bei besetzter Position 3 in Verbindungen der Formel XIV Gemische von Verbindungen der Formeln XII und XIII entstehen können, hauptsächlich jedoch Sulfonimide der Formel XII. Bei freier 3-Position in Verbindungen der Formel XIV entstehen fast ausschliesslich Verbindungen der Formel XIII. Die erwähnten Umsetzungen werden vorzugsweise unter den bei Reaktion A (II + III → I) angegebenen Bedingungen durchgeführt. Die Herstellung von Verbindungen der Formel XIV kann analog zu bekannten Methoden erfolgen, z.B. nach Tetrahedron 33 , 217 (1977) und DE- 3,001,579.

Die Ausgangsverbindungen der Formeln III, VIII, X, XI, XIII und XIV sind bekannt oder lassen sich analog zu den bekannten Vertretern herstellen.

Die Erfindung schliesst auch ein Verfahren zum prophylaktischen Schutz von Tieren gegen parasitäre Helminthen ein, das dadurch gekennzeichnet ist, dass man die Wirkstoffe der Formel I bzw. die Wirkstofformulierungen als Zusatz zum Futter oder zu den Tränken oder auch in fester oder flüssiger Form oral, durch Injektion oder mittels der Pour-on-Methode den Tieren appliziert.

Unter den bei Warmblütern vorkommenden Endoparasiten verursachen namentlich die Helminthen grosse Schäden. So können von diesen Parasiten befallene Tiere nicht nur ein verlangsamtes Wachstum, sondern erhebliche physiologische Beeinträchtigungen aufweisen, die sogar zur Mortalität führen können. Daher ist es von grosser Bedeutung, therapeutische Mittel zu entwickeln, die sich zur Bekämpfung von Helminthen und deren Entwicklungsstadien sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen sowie Pferden, Schweinen, Rotwild, Hunden, Katzen und Geflügel auftreten.

Die durch Helminthiasen verursachten Schäden können bei chronischem und vor allem bei epidemischem Auftreten der Wurmerkrankungen in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff "Helminthen" insbesondere parasitäre Würmer verstanden, die zu den Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen-species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38 , 1425-1426 (1977); Am. J. Vet. Res. 37 , 1515-1516 (1976); Am. J. Vet. Res. 38 , 807-808 (1977); Am. J. Vet. Res. 38 , 1247-1248 (1977)) besitzt zwar ein begrenztes anthelmintisches Wirkungsspektrum bei Wiederkäuern, seine Wirkung gegen Benzimidazol-resistente Nematoden und adulte Leberegel ist jedoch unzureichend, da vor allem die pathogenisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I nicht nur - wie bereits erwähnt - eine intensive anthelmintische Wirksamkeit mit breitem Wirkungsspektrum gegen Nematoden, Cestoden und Trematoden, sondern darüber hinaus zusätzlich eine günstige Warmblütertoxizität besitzen.

Die erfindungsgemässen neuen Wirkstoffe der Formel I sind beispielsweise zur Bekämpfung parasitärer Nematoden der Ordnungen (nach K.I. Skrajabin)
Rhabditida
Ascaridida
Spirurida
Trichocephalida
oder zur Bekämpfung von Cestoden der Ordnungen (nach Wardle & McLeod)
Cyclophyllidae
Pseudophyllidae
oder zur Bekämpfung von Trematoden der Ordnung
Digenea
bei Haus- und Nutztieren wie Rindern, Schafen, Ziegen, Pferden, Schweinen, Katzen, Hunden und Geflügel geeignet. Sie können den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die erfindungsgemäsen Mittel werden hergestellt, indem die Wirkstoffe der Formel I mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Fressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet: Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gela tine, lösliche Cellulosederivate, gewünschtenfalls unter Zusatz von oberflächenaktiven Stoffen wie ionischen oder nichtionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl scg. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt.

Häufig werden sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner können auch entsprechende Phosphate, wie z.B. Salze des Phosphorsäureesters eines p-Nonylphenol-(4-14)-Ethylenoxyd-Adduktes oder Phospholipide als Formulierungshilfsstoffe Anwendung finden.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1981; Stache, H., "Tensid-Taschenbuch", Carl Hanser Verlag, München/Wien, 1981

Als Bindemittel für Tabletten und Boli kommen chemisch abgewandelte, in Wasser oder Alkohol lösliche, polymere Naturstoffe in Frage, wie Stärke-, Cellulose- oder Proteinderivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.%, insbesondere 0,1 bis 95 Gew.-%, Wirkstoff der Formel I, und 99,9 bis 1 Gew.-%, insbesondere 99,9 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der

vorliegenden Erfindung.

Die nachfolgenden Beispiele dienen zur näheren Erläuterung der Erfindung, ohne dieselbe einzuschränken.

1. Herstellungsbeispiele

Nach Verfahren A

1.1. Herstellung von 5-Chlor-2-[(4-chlor-phenyl-sulfonyl)-amino]-benzoesäure-[4-(3-chlor-5-trifluormethyl-pyridyl-2-oxy)-anilid].

(a) 5-Chlor-2-nitro-benzoylchlorid

Ein Gemisch von 80,6 g 5-Chlor-2-nitro-benzoesäure und 58,2 ml Thionylchlorid wird während 2 Std. auf 90° C erhitzt. Anschliessend wird das überschüssige Thionylchlorid im Vakuum abdestilliert. Man erhält 87,1 g 5-Chlor-2-nitro-benzoylchlorid.

(b) 5-Chlor-2-nitro-benzoesäure[4-(3-chlor-5-trifluoromethyl-pyridyl-2-oxy)-anilid]

Zu einer Lösung von 5,8 g 5-Chlor-2-nitro-benzoylchlorid in 50 ml Methylenchlorid wird bei 0° C eine Lösung von 8,0 g 4-[3-Chlor-5-trifluormethyl-pyridyl-2-oxy]-anilin und 5,2 ml Triethylamin in 50 ml Methylenchlorid tropfenweise zugegeben. Nach 2 Std. Rühren bei Raumtemperatur wird etwa 80 % des Lösungsmittels in einem Rotationsverdampfer entfernt. Der erhaltene Kristallbrei wird mit 100 ml Diethylether verrührt, die anfallenden Kristalle werden mit 1N HCl-Lösung und $H_2O$ gewaschen und nochmals mit 100 ml Diethylether verrührt. Man erhält 9,6 g 5-Chlor-2-nitro-benzoesäure[4-(3-chlor-5-trifluoromethyl-pyridyl-2-oxy)-anilid] mit einem Smp. von 216-218° C.

(c) 2-Amino-5-chlor-benzoesäure-[4-(3-chlor-5-trifluormethyl-pyridyl-2-oxy)-anilid]

8,0 g 5-Chlor-2-nitro-benzoesäure-[4-(3-chlor-5-trifluormethyl-pyridyl-2-oxy)-anilid] werden in 90 ml Tetrahydrofuran gelöst und während 6 Stunden bei Raumtemperatur unter $H_2$-Atmosphäre in Gegenwart von 4 g 5 % Rhodium-Kohle hydriert. Das Reaktionsgemisch wird anschliessend filtriert und das Lösungsmittel abdestilliert. Das erhaltene Rohprodukt wird mit Diethylether verrührt. Man erhält 5,5 g 2-Amino-5-chlor-benzoesäure-[4-(3-chlor-5-trifluormethyl-pyridyl-2-oxy)-anilid] als weisse Kristalle (Smp. 180-184° C).

(d) 5-Chlor-2-[(4-chlorphenylsulfonyl)-amino]-benzoesäure-[4(3-chlor-5-trifluormethyl-pyridyl-2-oxy)-anilid]

Zu einer Lösung von 3,8 g 5-Chlor-2-amino-benzoesäure-[4-( 3-chlor-5-trifluormethyl-pyridyl-2-oxy)-anilid] in 8,6 ml Pyridin werden bei 0° C 2,0 g 4-Chlorbenzolsulfonsäurechlorid gegeben. Nach 16 Std. Rühren bei Raumtemperatur wird das Reaktionsgemisch mit 300 ml Methylenchlorid versetzt und die erhaltene Lösung anschliessend mit je 100 ml 1N HCl, $H_2O$, ges. $NaHCO_3$-Lösung und ges. NaCl-Lösung gewaschen. Die organische Phase wird dann mit $MgSO_4$ getrocknet und eingedampft. Das erhaltene Rohprodukt lässt sich durch Verrühren in Diethylether reinigen. Man erhält so 3,8 g 5-Chlor-2-[(4-chlorphenyl-sulfonyl)-amino]-benzoesäure-[4-(3-chlor-5-trifluormethyl-pyridyl-2-oxy)-anilid] als weisse Kristalle (Smp. 191-193° C).

Nach Verfahren B

2.1.Verfahren zur Herstellung von 3,5-Dichlor-2-[(4-chlor-phenylsulfonyl)-amino]-benzosäure-[4-(4-trifluormethyl-6-cyclopropylpyrimidin-2-yl-oxy)-anilid]

(a) 3,5-Dichlor-2-[di-(4-chlor-phenyl-sulfonyl)-amino]-benzoesäureethylester

Zu einer Lösung von 50,0 g 3,5-Dichloranthranilsäureethylester in 210 ml trockenem Pyridin werden bei Raumtemperatur portionenweise 138,0 g 4-Chlorbenzolsulfonsäurechlorid zugegeben. Das Reaktionsgemisch wird auf 80-90° C erhitzt und während 28 Stunden bei dieser Temperatur ausgerührt. Nach Abkühlen wird das Reaktionsgemisch auf Eis/Wasser gegossen und mit 2N Salzsäure angesäuert. Der klebrige, ölige Niederschlag wird in Diethylether gelöst, die Wasserphase abgetrennt. Die organische Phase wird mit Wasser und gesättigter Kochsalzlösung gewaschen. Nach Trocknen über Magnesiumsulfat wird der Ether abgedampft und die zurückbleibenden Kristalle in einem Gemisch von Diethylether/Hexan = 4/1 verrührt. Die abfiltrierten Kristalle wiegen nach dem Trocknen 87,3 g und entsprechen der Titelverbindung (Smp.: 148-150° C).

(b) 3,5-Dichlor-2-[(4-chlor-phenyl-sulfonyl)-amino]-benzoesäure

Eine Suspension von 27,0 g 3,5-Dichlor-2-[di-(4-chlor-phenyl-sulfonyl)-amino]-benzoesäure-ethylester, 139 ml 2N Natronlauge und 140 ml Ethanol wird während 6 Stunden am Rückfluss gekocht, wobei der Festkörper allmählich in Lösung geht. Nach Abkühlen des Reaktionsgemischs werden die ausgefallenen Kristalle filtriert und in 400 ml Wasser gelöst. Nach Zugabe von 400 ml Methanol wird mit 2N Salzsäure angesäuert, die ausgefallenen Kristalle werden abfiltriert. Man erhält 12,3 g der Titelverbindung (Smp. 188-190° C).

(c) 3,5-Dichlor-2-[(4-chlor-phenyl-sulfonyl)-amino]-benzosäure-[4-(4-trifluormethyl-6-cyclopropylpyrimidin-2-yl-oxy)-anilid]

4,0 g 3,5-Dichlor-2-[4-chlor-phenyl-sulfonyl)-amino]-benzoesäure werden in 10 ml Thionylchlorid suspendiert. Das Reaktionsgemisch wird während 3 Stunden am Rückfluss gekocht. Nach Abdestillieren des überschüssigen Thionylchlorids wird der Rückstand zweimal aus 20 ml Toluol eingedampft. Das so roh erhaltene, feste Säurechlorid wird in 30 ml Methylenchlorid gelöst. Zu dieser Lösung wird unter Eis/Wasser-Kühlung langsam eine Lösung von 3,3 g 4-(4-Trifluormethyl)-6-cyclopropyl-pyrimidin-2-yl-oxy)anilin [Smp. 75-78° C], 2 ml Triethylamin und 20 ml Methylenchlorid zugetropft. Nach 24 Stunden bei Raumtemperatur wird der ausgefallene Festkörper abfiltriert und mit wenig Methylenchlorid gewaschen. Das Rohprodukt wird mit 2N Salzsäure und Wasser gewaschen, dann getrocknet und mit 50 ml Diethylether verrührt. Man erhält 2,27 g der Titelverbindung (Smp. 273-276° C). Durch Aufarbeitung der eingedampften Mutterlaugen in oben erwähnter Weise lassen sich weitere 2,06 g der Titelverbindung isolieren.

Analog zu den beschriebenen Arbeitsweisen lassen sich auch die nachfolgend aufgelisteten Verbindungen herstellen. Die Listen haben keinen limitierenden Charakter.

Tabelle 1: Verbindungen der Formel Ia

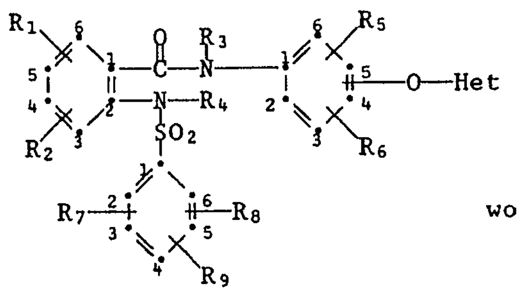

worin $R_9$ für Wasserstoff steht.

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.1 | 5-Cl | H | H | H | H | H | 4-Cl | H | 4 | 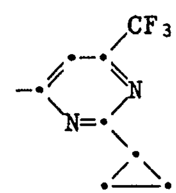 | 199-201 | A |
| 1.2 | 5-Cl | H | H | H | H | H | 4-Cl | H | 4 | 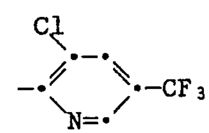 | 191-193 | A |
| 1.3 | 5-Cl | H | H | H | H | H | 4-Cl | H | 4 | 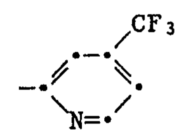 | 162-165 | A |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.4 | 5-Cl | H | H | H | H | H | 4-Cl | H | 4 | 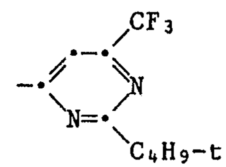 | 203-204 | A |
| 1.5 | 5-Cl | 3-Cl | H | H | H | H | 4-Cl | H | 4 | 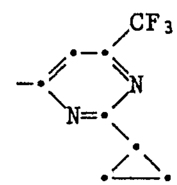 | 273-276 | B |
| 1.6 | 5-Cl | 3-Cl | H | H | H | H | 4-Cl | H | 4 | 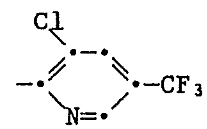 | 285-286 | B |
| 1.7 | 5-$SCH_3$ | H | H | H | H | H | 4-Cl | H | 4 | 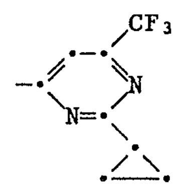 | 167-170 | A |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.8 | 5-Cl | H | H | H | H | H | $4-NO_2$ | H | 4 | 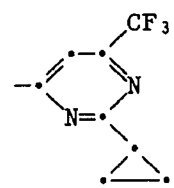 | | A |
| 1.9 | $6-CH_3$ | $3-OCH_3$ | H | H | H | H | 4-Cl | H | 4 | 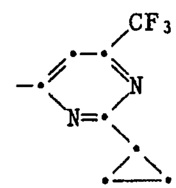 | | A |
| 1.10 | 5-Cl | H | H | H | H | H | 4-Cl | H | 4 | 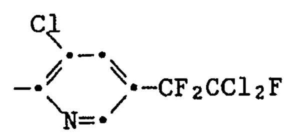 | | A |
| 1.11 | 5-Cl | 3-Cl | H | H | H | H | 4-Cl | H | 4 | 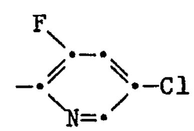 | 258-260 | B |
| 1.12 | 5-Cl | 3-Cl | H | H | H | H | 4-Cl | H | 4 | 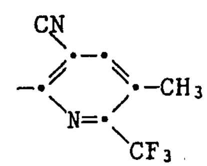 | 309-312 | B |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.13 | 5-Cl | 3-Cl | H | H | H | H | 4-Cl | H | 4 | 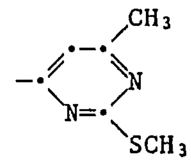 | 264-265 | B |
| 1.14 | 5-Cl | 3-Cl | H | H | H | H | 4-Cl | H | 4 | 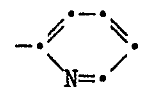 | 228-231 | B |
| 1.15 | 5-Cl | 3-Cl | H | H | 2-iso-propyl | H | 4-Cl | H | 4 | 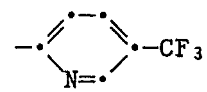 | 250-252 | B |
| 1.16 | 5-Cl | 3-Cl | H | H | 2-$CH_3$ | 6-$CH_3$ | 4-Cl | H | 4 | 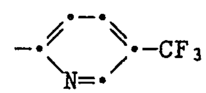 | 282-285 | B |
| 1.17 | 5-Cl | 3-Cl | H | H | H | H | 4-Cl | H | 4 | 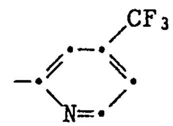 | 264-266 | B |
| 1.18 | 5-Cl | 3-Cl | H | H | H | H | 4-Cl | H | 4 | 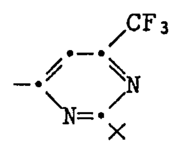 | 256-259 | B |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.19 | 5-Cl | H | H | H | H | H | 4-Cl | H | 4 | 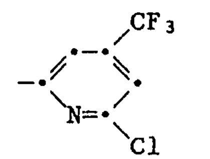 | 175-177 | B |
| 1.20 | 5-$CF_3$ | H | H | H | H | H | 4-Cl | H | 4 | 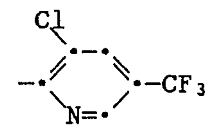 | | B |
| 1.21 | 3-$CF_3$ | H | H | H | H | H | 4-Cl | H | 4 | 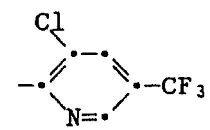 | 278-281 | A |
| 1.22 | 4-$OCH_3$ | 6-$CH_3$ | H | H | H | H | 4-Cl | H | 4 | 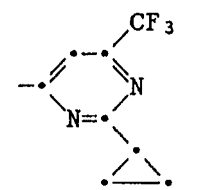 | | B |
| 1.23 | 5-S(O)$CH_3$ | H | H | H | H | H | 4-Cl | H | 4 | 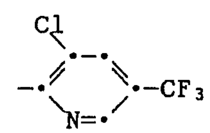 | | Nachoxidation |

<u>Tabelle 1</u> (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.24 | 5-$SO_2CH_3$ | H | H | H | H | H | 4-Cl | H | 4 | 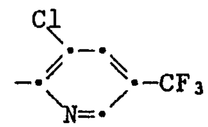 | | Nachoxidation |
| 1.25 | 5-$SCH_3$ | H | H | H | H | H | 4-Cl | H | 4 | 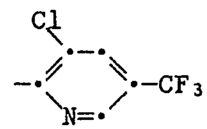 | 158-161 | A |
| 1.26 | 5-F | H | H | H | H | H | 4-Cl | H | 4 | 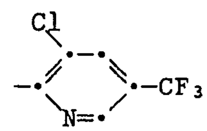 | 218-220 | A |
| 1.27 | 5-F | 3-F | H | H | H | H | 4-Cl | H | 4 | 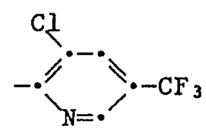 | 278-279 | A |
| 1.28 | 5-Cl | 3-Cl | H | H | H | H | 4-Cl | H | 4 | 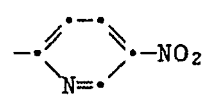 | 258-261 | B |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.29 | 5-Cl | 3-Cl | H | H | 4-$CH_3$ | H | 4-Cl | H | 3 | 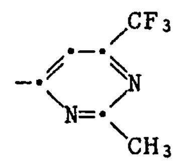 | 244-246 | B |
| 1.30 | 5-Cl | H | $CH_3$ | H | H | H | 4-Cl | H | 4 | 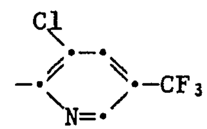 | 169-170 | B |
| 1.31 | 5-Cl | H | H | H | 2-F | H | 4-Cl | H | 4 | 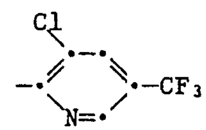 | 145-147 | B |
| 1.32 | 5-Cl | H | H | H | 2-F | H | 4-Cl | H | 4 | 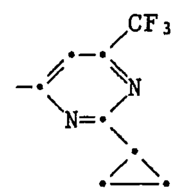 | 155-157 | B |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.33 | 5-Cl | H | H | $CH_3$ | H | H | 4-Cl | H | 4 | 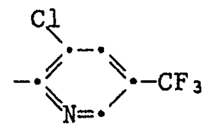 | 176-178 | A |
| 1.34 | 5-Cl | H | H | H | H | H | 4-Cl | 3-Cl | 4 | 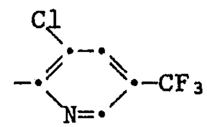 | | A |
| 1.35 | 5-Cl | H | H | H | 4-Cl | H | 4-Cl | H | 3 | 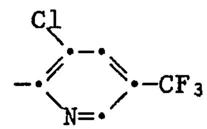 | 188-190 | A |
| 1.36 | 5-Cl | H | H | H | 4-Cl | H | 4-F | H | 3 | 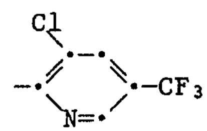 | 214-218 | A |
| 1.37 | 5-$OCH_3$ | H | H | H | H | H | 4-Cl | H | 4 | 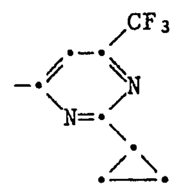 | | B |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.38 | 5-Cl | H | H | H | H | H | $4\text{-}OCH_3$ | H | 4 | 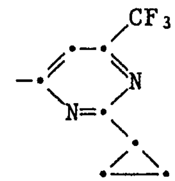 | | A |
| 1.39 | 4-Cl | H | H | H | H | H | 4-Cl | H | 4 | 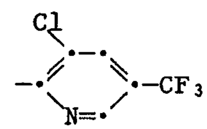 | 197-200 | B |
| 1.40 | $6\text{-}CH_3$ | H | H | H | H | H | $4\text{-}CH_3$ | H | 4 | 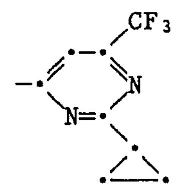 | 207-211 | B |
| 1.41 | 5-F | H | H | H | H | H | 4-Cl | H | 4 | 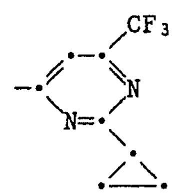 | 231-233 | A |

Tabelle 1 (Fortsetzung)

| Verb. Nr. | $R_1$ | $R_2$ | $R_3$ | $R_4$ | $R_5$ | $R_6$ | $R_7$ | $R_8$ | Position von Het | Het | Smp. [°C] | Herstellungs-variante |
|---|---|---|---|---|---|---|---|---|---|---|---|---|
| 1.42 | 5-F | 3-F | H | H | H | H | 4-Cl | H | 4 | 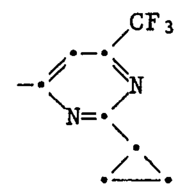 | 245-247 | A |
| 1.43 | 5-Cl | 4-Cl | H | H | H | H | 4-Cl | H | 4 | 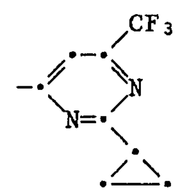 | | |

Tabelle 2 Zwischenprodukte der Formel

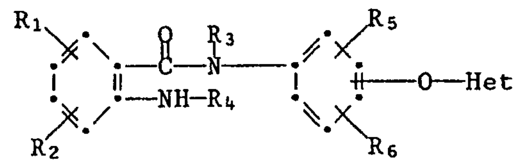

| Verb. Nr. | $R_1$ | $R_2$ | $R_3$ | $R_4$ | $R_6$ | $R_5$ | Position von Het | Het | Smp. [°C] |
|---|---|---|---|---|---|---|---|---|---|
| 2.1 | 5-Cl | H | H | H | H | H | 4 | 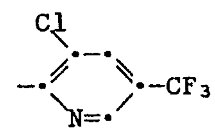 | 180-184 |
| 2.2 | 5-Cl | H | H | H | H | H | 4 | 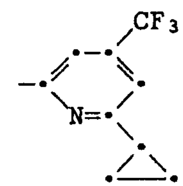 | 175-177 |
| 2.3 | 5-Cl | H | H | H | H | H | 4 | 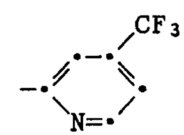 | 182-183 |
| 2.4 | 5-Cl | H | H | H | H | H | 4 | 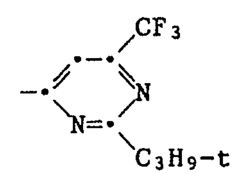 | 173-174 |

Tabelle 2 (Fortsetzung)

| Verb. Nr. | $R_1$ | $R_2$ | $R_3$ | $R_4$ | $R_6$ | $R_5$ | Position von Het | Het | Smp. [°C] |
|---|---|---|---|---|---|---|---|---|---|
| 2.5 | 5-Cl | H | H | $CH_3$ | H | H | 4 | 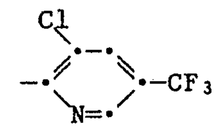 | 190-192 |
| 2.6 | 4-Cl | H | H | H | H | H | 4 | 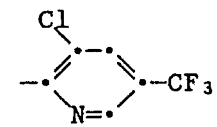 | 218-220 |
| 2.7 | 5-$SCH_3$ | H | H | H | H | H | 4 | 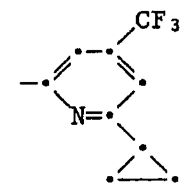 | 159-162 |
| 2.8 | 5-$SCH_3$ | H | H | H | H | H | 4 | 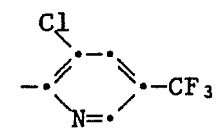 | 157-161 |
| 2.9 | 3-$CF_3$ | H | H | H | H | H | 4 | 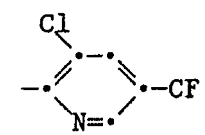 | 165-167 |
| 2.10 | 5-Cl | H | H | H | 4-Cl | H | 3 | 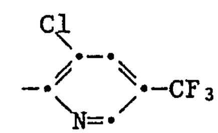 | 193-194 |

Tabelle 2 (Fortsetzung)

| Verb. Nr. | $R_1$ | $R_2$ | $R_3$ | $R_4$ | $R_6$ | $R_5$ | Position von Het | Het | Smp. [°C] |
|---|---|---|---|---|---|---|---|---|---|
| 2.11 | 5-F | H | H | H | H | H | 4 | 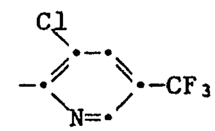 | 166-168 |
| 2.12 | 5-F | H | H | H | H | H | 4 | 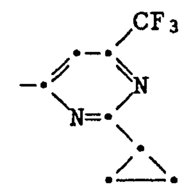 | 168-170 |
| 2.13 | 5-F | 3-F | H | H | H | H | 4 | 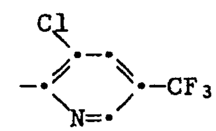 | 196-198 |
| 2.14 | 5-F | 3-F | H | H | H | H | 4 | 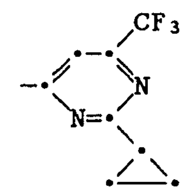 | 189-190 |

Tabelle 3 Zwischenprodukte der Formel

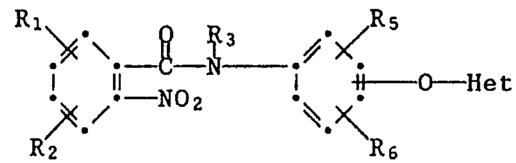

| Verb. Nr. | $R_1$ | $R_2$ | $R_3$ | $R_5$ | $R_6$ | Position von Het | Het | Smp. [°C] |
|---|---|---|---|---|---|---|---|---|
| 3.1 | 5-Cl | H | H | H | H | 4 | 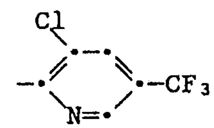 | 216-218 |
| 3.2 | 5-Cl | H | H | H | H | 4 | 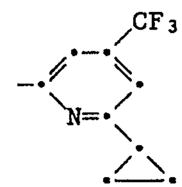 | 188-192 |
| 3.3 | 5-Cl | H | H | H | H | 4 | 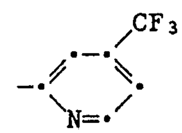 | 203-205 |
| 3.4 | 5-Cl | H | H | H | H | 4 | 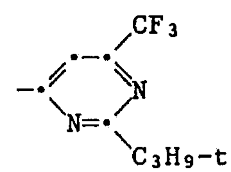 | 202-203 |

Tabelle 3 (Fortsetzung)

| Verb. Nr. | $R_1$ | $R_2$ | $R_3$ | $R_5$ | $R_6$ | Position von Het | Het | Smp. [°C] |
|---|---|---|---|---|---|---|---|---|
| 3.5 | 4-Cl | H | H | H | H | 4 | 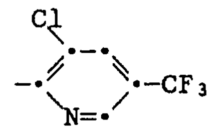 | 214-216 |
| 3.6 | 4-Cl | H | H | H | H | 4 | 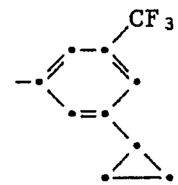 | 195-198 |
| 3.7 | 5-Cl | H | H | H | 4-Cl | 3 | 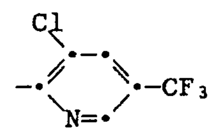 | 168-170 |
| 3.8 | 5-$SCH_3$ | H | H | H | H | 4 | 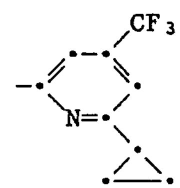 | 204-207 |
| 3.9 | 5-$SCH_3$ | H | H | H | H | 4 | 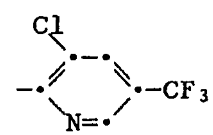 | 221-224 |

Tabelle 3 (Fortsetzung)

| Verb. Nr. | $R_1$ | $R_2$ | $R_3$ | $R_5$ | $R_6$ | Position von Het | Het | Smp. [°C] |
|---|---|---|---|---|---|---|---|---|
| 3.10 | 5-F | H | H | H | H | 4 | 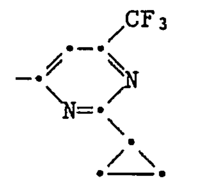 | 181-182 |
| 3.11 | 5-F | H | H | H | H | 4 | 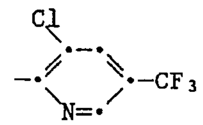 | 206-207 |
| 3.12 | 5-F | 3-F | H | H | H | 4 | 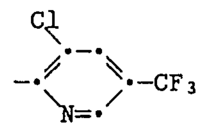 | 217-218 |
| 3.13 | 5-F | 3-F | H | H | H | 4 | 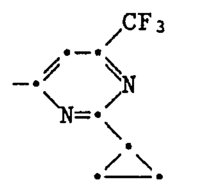 | 219-220 |

2. Formulierungsbeispiele (% = Gewichtsprozent)

| 2.1. Emulsions-Konzentrate | a) | b) | c) |
|---|---|---|---|
| Wirkstoff aus Tabelle I | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus Tabelle I | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenze 160-190 °C) | - | - | 94 % | - |
| (MG = Molekulargewicht) | | | | |

Die Lösungen sind zur Anwendung in Form kleinster Tropfen geeignet.

| 2.3. Granulate | a) | b) |
|---|---|---|
| Wirkstoff der Tabelle I | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

| 2.4. Stäubemittel | a) | b) |
|---|---|---|
| Wirkstoff der Tabelle I | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| 2.5. In Wasser dispergierbare Pulvermischung | a) | b) | c) |
|---|---|---|---|
| Wirkstoff aus Tabelle I | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions-Konzentrat | a) | b) | c) |
|---|---|---|---|
| Wirkstoff aus Tabelle I | 10 % | 8 % | 60 % |
| Octylphenolpolyethylenglykolether (4-5 Mol Ethylenoxid) | 3 % | 3 % | 2 % |
| Ricinusölpolyethylenglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus Tabelle I | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8. Granulat | |
|---|---|
| Wirkstoff aus Tabelle I | 10 % |
| Na-Ligninsulfonat | 2 % |
| Carboxymethylcellulose | 1% |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Granulat | |
|---|---|
| Wirkstoff aus Tabelle I | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus Tabelle I | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentrationhergestellt werden können.

| 2.11. Pellets bzw. Boli | |
|---|---|
| I Wirkstoff der Tabelle I | 33,0 % |
| Methylcellulose | 0,80 % |
| Kieselsäure hochdispers | 0,80 % |
| Maisstärke | 8,40 % |
| II Milchzucker krist. | 22,50 % |
| Maisstärke | 17,00 % |
| mikrokrist. Cellulose | 16,50 % |
| Magnesiumstearat | 1,00 % |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.
II Alle 4 Hilfsstoffe gut mischen.
III Phasen I und II mischen und zu Pellets oder Boli verpressen.

## 3. Biologische Beispiele

Die anthelmintische Wirksamkeit wird anhand folgender Versuche demonstriert:

### 3.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird nur mit einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Als Suspension eingesetzte Wirkstoffe aus Tabelle I bewirken bei Schafen in einer Dosis, welche bei 50 mg/kg Körpergewicht oder tiefer liegt, eine Reduzierung des Nematodenbefalls um 90 % oder mehr, verglichen mit unbehandelten, aber infizierten Vergleichsgruppen. Verbindungen der Formel I wie z.B. Nr. 1.1 und 1.2 ergeben selbst bei einer Dosis von 20 mg/kg Körpergewicht eine Reduzierung des Nematodenbefalls um mehr als 90 %.

### 3.2. Versuch an mit Fasciola hepatica infizierten Schafen

Der Wirkstoff wird in Form einer Suspension mit einer Magensonde oder durch Pansen-Injektion Schafen verabreicht, die vorher mit Fasciola hepatica künstlich infiziert wurden. Pro Versuch resp. pro Dosis werden 3 Tiere verwendet. Jedes Tier wird mit nur einer einzigen Dosis behandelt.

Die erste Evaluierung erfolgt dadurch, dass die Anzahl der vor und nach der Behandlung im Kot der Schafe ausgeschiedenen Wurmeier verglichen wird.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der

Unterschied der in den beiden Gruppen festgestellten Anzahl von Leberegeln ergibt den Wirkungsgrad des geprüften Wirkstoffs.

Suspensionen mit Wirkstoffen aus Tabelle I erbringen gute Resultate, d.h., eine Abnahme des Leberegelbefalls um mindestens 90 % bei einer Dosis von 50 mg/kg Körpergewicht oder tiefer.

## Ansprüche

1. Verbindungen der Formel I

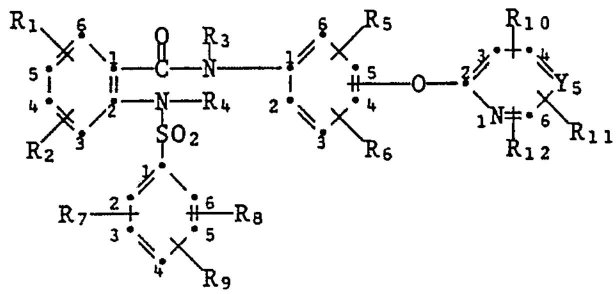

worin

$R_1$ für Wasserstoff, Halogen, $C_1$-$C_2$-Alkyl, $C_1$-$C_2$-Thioalkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht;

$R_2$ für Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy steht;

$R_3$ Wasserstoff oder $C_1$-$C_2$-Alkyl bedeutet;

$R_4$ Wasserstoff oder $C_1$-$C_2$-Alkyl bedeutet;

$R_5$ für Wasserstoff, Halogen oder $C_1$-$C_5$-Alkyl steht;

$R_6$ für Wasserstoff, Halogen oder $C_1$-$C_5$-Alkyl steht;

$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;

$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_0$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;

$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;

$R_{12}$ für Wasserstoff oder Halogen steht; und

Y =CH- oder =N- repräsentiert; unter Einschluss ihrer physiologisch verträglichen Säureadditionssalze.

2. Verbindungen nach Anspruch 1, worin $R_1$ für Wasserstoff, Halogen, $C_1$-$C_2$-Alkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht; und $R_2$ bis $R_{12}$ und Y wie in Anspruch 1 definiert sind.

3. Verbindungen der Formel I nach Anspruch 1, worin

$R_1$, $R_2$, $R_3$, $R_4$, $R_7$, $R_8$, $R_9$, $R_{12}$ und Y die unter Formel I angegebenen Bedeutungen haben;

$R_5$ für Wasserstoff, Halogen oder $C_1$-$C_3$-Alkyl steht;

$R_6$ für Wasserstoff, Halogen oder $C_1$-$C_3$-Alkyl steht;

$R_{10}$ für Wasserstoff, Halogen, $C_1$-$C_4$-Alkyl, $C_1$-$C_2$-Haloalkyl, $C_1$-$C_2$-Alkylthio, Cyclopropyl oder Cyano steht; und

$R_{11}$ für Wasserstoff, Halogen, $C_1$-$C_4$-Alkyl, $C_1$-$C_2$-Haloalkyl, $C_1$-$C_2$-Alkylthio oder Cyclopropyl steht; unter Einschluss ihrer physiologisch verträglichen Säureadditionssalze.

4. Verbindungen der Formel I nach Anspruch 1, worin

$R_1$ für Wasserstoff, Halogen, Methyl, Methoxy oder Thiomethyl steht;

$R_2$ Wasserstoff, Methyl oder Halogen bedeutet;

$R_3$ Wasserstoff, Methyl oder Ethyl bedeutet;

$R_4$ Wasserstoff , Methyl oder Ethyl bedeutet;

$R_5$ für Wasserstoff, Halogen oder $C_1$-$C_4$-Alkyl steht;

$R_6$ für Wasserstoff, Halogen oder $C_1$-$C_4$-Alkyl steht;

$R_7$ Wasserstoff, Halogen, Methyl, Methoxy, $CF_3$ oder Nitro bedeutet; $R_8$ für Wasserstoff oder Halogen steht;

$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ und $R_{11}$ unabhängig voneinander für Wasserstoff, Halogen, $C_1$-$C_4$-Alkyl, Methylthio, Trifluormethyl, $CF_2CCl_2F$, Cyclopropyl oder Cyano stehen;
$R_{12}$ für Wasserstoff oder Halogen steht; und
Y für =CH- oder =N- steht, wobei der Pyridinoxy- oder Pyrimidinoxy-Substituent über die 3- oder 4-Position des Phenylrings gebunden ist.

5. Verbindungen der Formel I nach Anspruch 1, worin $R_1$ für 5-Halogen oder 4-Halogen steht; $R_2$ Wasserstoff bedeutet;
$R_3$ Wasserstoff oder $C_1$-$C_2$-Alkyl bedeutet;
$R_4$ Wasserstoff oder $C_1$-$C_2$-Alkyl bedeutet;
$R_5$ für Wasserstoff, Halogen oder $C_1$-$C_5$-Alkyl steht;
$R_6$ für Wasserstoff, Halogen oder $C_1$-$C_5$-Alkyl steht;
$R_7$ Wasserstoff, Halogen, $C_1$-$C_2$-Alkyl, Nitro $C_1$-$C_2$-Haloalkyl, $C_1$-$C_2$-Haloalkoxy oder $C_1$-$C_2$-Alkoxy bedeutet;
$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;
$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;
$R_{12}$ für Wasserstoff oder Halogen steht; und
Y =CH- oder =N- repräsentiert; unter Einschluss ihrer physiologisch verträglichen Säureadditionssalze.

6. Verbindungen der Formel I nach Anspruch 5, worin $R_1$ für 5-Chlor oder 4-Chlor steht;
$R_2$ Wasserstoff bedeutet,
$R_3$ Wasserstoff, Methyl oder Ethyl bedeutet;
$R_4$ Wasserstoff , Methyl oder Ethyl bedeutet;
$R_5$ für Wasserstoff, Halogen oder $C_1$-$C_4$-Alkyl steht;
$R_6$ für Wasserstoff, Halogen oder $C_1$-$C_4$-Alkyl steht;
$R_7$ Wasserstoff, Halogen, Methyl, Methoxy, $CF_3$ oder Nitro bedeutet;
$R_8$ für Wasserstoff oder Halogen steht;
$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;
$R_{10}$ und $R_{11}$ unabhängig voneinander für Wasserstoff, Halogen, $C_1$-$C_4$-Alkyl, Methylthio, Trifluormethyl, $CF_2CCl_2F$, Cyclopropyl oder Cyano stehen; $R_{12}$ für Wasserstoff oder Halogen steht; und
Y für =CH- oder =N- steht, wobei der Pyridinoxy- oder Pyrimidinoxy-Substituent über die 3- oder 4-Position des Phenylrings gebunden ist.

7. Eine Verbindung der Formel I nach Anspruch 1, ausgewählt aus der Reihe:
5-Chlor-2-[(4-chlor-phenyl-sulfonyl)-amino]-benzoesäure-[4-(4-trifluormethyl-6-cyclopropyl-pyrimidin-2-yl-oxy)-anilid];
5-Chlor-2-[(4-chlor-phenyl-sulfonyl)-amino]-benzoesäure-[4-(3-chlor-5-trifluormethyl-pyridin-2-yl-oxy)-anilid];
5-Chlor-2-[(4-chlor-phenyl-sulfonyl)-amino]-benzoesäure-[4-(4-trifluormethyl-pyridin-2-yl-oxy)-anilid];
5-Chlor-2-[(4-chlor-phenyl-sulfonyl)-amino]-benzoesäure-[4-(4-trifluormethyl-6-tert.butyl-pyrimidin-2-yl-oxy)-anilid];
3,5-Dichlor-2-[(4-chlor-phenyl-sulfonyl)-amino]-benzoesäure-[4-(4-trifluormethyl-6-cyclopropyl-pyrimidin-2-yl-oxy)-anilid];
3,5-Dichlor-2-[(4-chlor-phenyl-sulfonyl)-amino]-benzoesäure-[4-(5-trifluormethyl-pyridin-2-yl-oxy)-anilid].

8. Die Verbindung 4-Chlor-2-[(4-chlor-phenyl-sulfonyl)-amino]-benzoesäure-[4-(3-chlor-5-trifluormethyl-pyridin-2-yl-oxy)-anilid] nach Anspruch 1.

9. Verfahren zur Herstellung einer Verbindung der Formel I nach Anspruch 1, dadurch gekennzeichnet, dass man

(A) eine Verbindung der Formel II

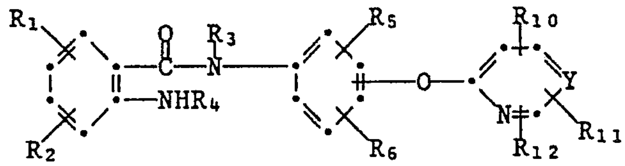

(II)

+

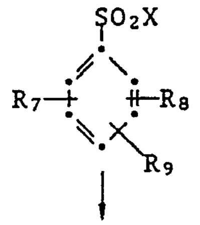

(III)

(I)

mit einem Benzolsulfonsäurehalogenid der Formel III, oder
(B) eine Verbindung der Formel IV

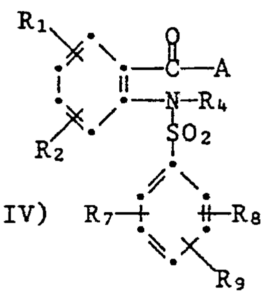

(IV)

+

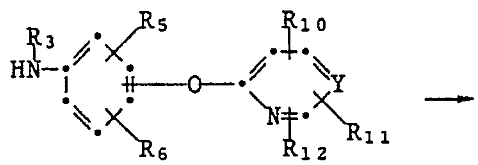

(V)

(I)

mit einer Verbindung der Formel V umsetzt, wobei die Substituenten $R_1$ bis $R_{12}$ sowie Y in den Formeln II, III, IV und V die unter Formel I angegebenen Bedeutungen haben, X in Formel III für Halogen, vorzugsweise Chlor oder Brom, insbesondere Chlor steht und A in Formel IV für Halogen (bevorzugt Chlor oder Brom), OH oder eine zur Reaktion mit einer Aminogruppe befähigte Abgangsgruppe darstellt und gegebenenfalls eine Verbindung der Formel II worin $R_3$ und $R_4$ oder $R_3$ oder $R_4$ für Wasserstoff stehen, nachalkyliert.

10. Anthelmintisches Mittel dadurch gekennzeichnet, dass es neben physiologisch verträglichen Formulierungshilfsstoffen eine Verbindung der Formel I nach einem der Ansprüche 1 bis 8 enthält.

11. Verfahren zur Bekämpfung von Helminthen beim Warmblüter, dadurch gekennzeichnet, dass man eine Verbindung der Formel I nach einem der Ansprüche 1 bis 8 in einer anthelmintisch aktiven Menge prophylaktisch oder kurativ an den Warmblüter verabreicht.

12. Verbindungen der Formel I nach einem der Ansprüche 1 bis 8 zur Verwendung in einem Verfahren zur Bekämpfung von Helminthen beim Warmblüter.

13. Verwendung von Verbindungen der Formel I nach einem der Ansprüche 1 bis 8 zur Herstellung von Anthelmintika.

14. Verbindungen der Formel

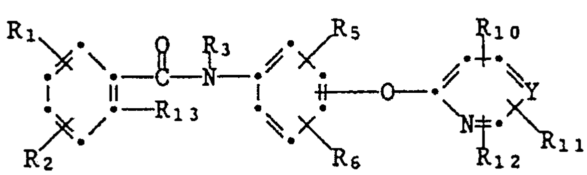

worin $R_1$, $R_2$, $R_3$, $R_5$, $R_6$, $R_{10}$, $R_{11}$ und $R_{12}$ wie in Anspruch 1 definiert sind und $R_{13}$ für $-NO_2$ oder $-NHR_4$ steht, wobei $R_4$ ebenfalls wie in Anspruch 1 definiert ist.

Patentansprüche für folgende Vertragsstaaten: ES, GR

1. Verfahren zur Herstellung einer Verbindung der Formel I

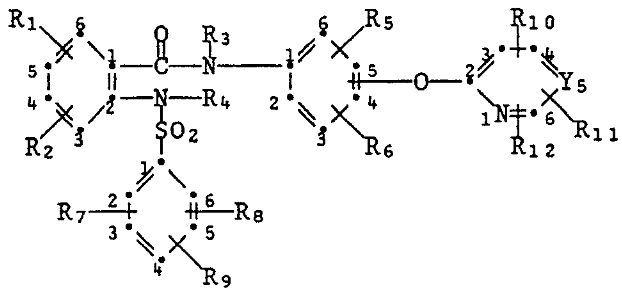

worin

$R_1$ für Wasserstoff, Halogen, $C_1$-$C_2$-Alkyl, $C_1$-$C_2$-Thioalkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht;

$R_2$ für Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy steht;

$R_3$ Wasserstoff oder $C_1$-$C_2$-Alkyl bedeutet;

$R_4$ Wasserstoff oder $C_1$-$C_2$-Alkyl bedeutet;

$R_5$ für Wasserstoff, Halogen oder $C_1$-$C_5$-Alkyl steht;

$R_6$ für Wasserstoff, Halogen oder $C_1$-$C_5$-Alkyl steht;

$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;

$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;

$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;

$R_{12}$ für Wasserstoff oder Halogen steht; und

Y =CH- oder =N- repräsentiert; unter Einschluss ihrer physiologisch verträglichen Säureadditionssalze, dadurch gekennzeichnet, dass man

(A) eine Verbindung der Formel II

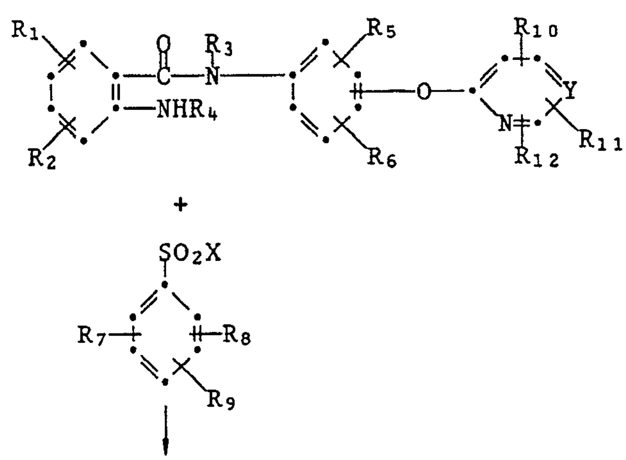

(II)

+

(III)

(I)

mit einem Benzolsulfonsäurehalogenid der Formel III, oder

(B) eine Verbindung der Formel IV

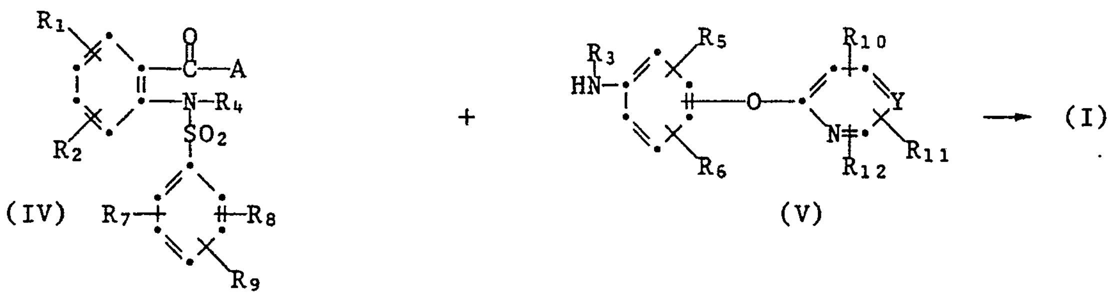

(IV) + (V) → (I).

mit einer Verbindung der Formel V umsetzt, wobei die Substituenten $R_1$ bis $R_{12}$ sowie Y in den Formeln II, III, IV und V die unter Formel I angegebenen Bedeutungen haben, X in Formel III für Halogen, vorzugsweise Chlor oder Brom, insbesondere Chlor steht und A in Formel IV für Halogen (bevorzugt Chlor oder Brom), OH oder eine zur Reaktion mit einer Aminogruppe befähigte Abgangsgruppe darstellt und gegebenenfalls eine Verbindung der Formel II worin $R_3$ und $R_4$ oder $R_3$ oder $R_4$ für Wasserstoff stehen, nachalkyliert.

2. Verfahren nach Anspruch 1, dadurch gekennzeichnet, dass es sich um Verbindungen der Formel I handelt, worin

$R_1$ für Wasserstoff, Halogen, $C_1$-$C_2$-Alkyl, $C_1$-$C_2$-Haloalkyl, Nitro, $C_1$-$C_2$-Alkoxy oder für die Gruppe $SO_nR$ steht, worin R $C_1$-$C_2$-Alkyl oder Phenyl bedeutet und n für 0, 1 oder 2 steht; und $R_2$ bis $R_{12}$ und Y die in Anspruch 1 angegebenen Bedeutungen haben.

3. Verfahren nach Anspruch 1, dadurch gekennzeichnet, dass es sich um eine Verbindung der Formel I handelt, worin

$R_1$, $R_2$, $R_3$, $R_4$, $R_7$, $R_8$, $R_9$, $R_{12}$ und Y die unter Formel I angegebenen Bedeutungen haben;

$R_5$ für Wasserstoff, Halogen oder $C_1$-$C_3$-Alkyl steht;

$R_6$ für Wasserstoff, Halogen oder $C_1$-$C_3$-Alkyl steht;

$R_{10}$ für Wasserstoff, Halogen, $C_1$-$C_4$-Alkyl, $C_1$-$C_2$-Haloalkyl, $C_1$-$C_2$-Alkylthio, Cyclopropyl oder Cyano steht; und

$R_{11}$ für Wasserstoff, Halogen, $C_1$-$C_4$-Alkyl, $C_1$-$C_2$-Haloalkyl, $C_1$-$C_2$-Alkylthio oder Cyclopropyl steht; unter Einschluss ihrer physiologisch verträglichen Säureadditionssalze.

4. Verfahren nach Anspruch 2, dadurch gekennzeichnet, dass es sich um eine Verbindung der Formel I handelt, worin

$R_1$ für Wasserstoff, Halogen, Methyl, Methoxy oder Thiomethyl steht;

$R_2$ Wasserstoff, Methyl oder Halogen bedeutet;

$R_3$ Wasserstoff, Methyl oder Ethyl bedeutet;

$R_4$ Wasserstoff , Methyl oder Ethyl bedeutet;

$R_5$ für Wasserstoff, Halogen oder $C_1$-$C_4$-Alkyl steht;

$R_6$ für Wasserstoff, Halogen oder $C_1$-$C_4$-Alkyl steht;

$R_7$ Wasserstoff, Halogen, Methyl, Methoxy, $CF_3$ oder Nitro bedeutet; $R_8$ für Wasserstoff oder Halogen steht;

$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_{10}$ und $R_{11}$ unabhängig voneinander für Wasserstoff, Halogen, $C_1$-$C_4$-Alkyl, Methylthio, Trifluormethyl, $CF_2CCl_2F$, Cyclopropyl oder Cyano stehen;

$R_{12}$ für Wasserstoff oder Halogen steht; und

Y für =CH- oder =N- steht, wobei der Pyridinoxy- oder Pyrimidinoxy-Substituent über die 3- oder 4-Position des Phenylrings gebunden ist.

5. Verfahren nach Anspruch 1, dadurch gekennzeichnet, dass es sich um eine Verbindung der Formel I handelt, worin $R_1$ für 5-Halogen oder 4-Halogen steht; $R_2$ Wasserstoff bedeutet;

$R_3$ Wasserstoff oder $C_1$-$C_2$-Alkyl bedeutet;

$R_4$ Wasserstoff oder $C_1$-$C_2$-Alkyl bedeutet;

$R_5$ für Wasserstoff, Halogen oder $C_1$-$C_5$-Alkyl steht;

$R_6$ für Wasserstoff, Halogen oder $C_1$-$C_5$-Alkyl steht;

$R_7$ Wasserstoff, Halogen, $C_1$-$C_2$-Alkyl, Nitro, $C_1$-$C_2$-Haloalkyl, $C_1$-$C_2$-Haloalkoxy oder $C_1$-$C_2$-Alkoxy bedeutet;

$R_8$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_{10}$ für Wasserstoff, Halogen, $C_1$-$C_6$-Alkyl, $C_1$-$C_2$-Haloalkyl, $C_1$-$C_2$-Alkylthio, $C_3$-$C_6$-Cycloalkyl oder Cyano steht;

$R_{11}$ für Wasserstoff, Halogen, $C_1$-$C_6$-Alkyl, $C_1$-$C_2$-Haloalkyl, $C_1$-$C_2$-Alkylthio, oder $C_3$-$C_6$-Cycloalkyl steht;

$R_{12}$ für Wasserstoff oder Halogen steht; und

Y =CH- oder =N- repräsentiert; unter Einschluss ihrer physiologisch verträglichen Säureadditionssalze.

6. Verfahren nach Anspruch 5, dadurch gekennzeichnet, dass es sich um eine Verbindung der Formel I handelt, worin $R_1$ für 5-Chlor oder 4-Chlor steht;

$R_2$ Wasserstoff bedeutet,

$R_3$ Wasserstoff, Methyl oder Ethyl bedeutet;

$R_4$ Wasserstoff , Methyl oder Ethyl bedeutet;

$R_5$ für Wasserstoff, Halogen oder $C_1$-$C_4$-Alkyl steht;

$R_6$ für Wasserstoff, Halogen oder $C_1$-$C_4$-Alkyl steht;

$R_7$ Wasserstoff, Halogen, Methyl, Methoxy, $CF_3$ oder Nitro bedeutet; $R_8$ für Wasserstoff oder Halogen steht;

$R_9$ Wasserstoff, Halogen, $C_1$-$C_2$-Alkyl, $C_1$-$C_2$-Haloalkyl, $C_1$-$C_2$-Haloalkoxy oder $C_1$-$C_2$-Alkoxy bedeutet;

$R_{10}$ und $R_{11}$ unabhängig voneinander für Wasserstoff, Halogen, $C_1$-$C_4$-Alkyl, Methylthio, Trifluormethyl, $CF_2CCl_2F$, Cyclopropyl oder Cyano stehen;

$R_{12}$ für Wasserstoff oder Halogen steht; und

Y für =CH- oder =N- steht, wobei der Pyridinoxy- oder Pyrimidinoxy-Substituent über die 3- oder 4-Position des Phenylrings gebunden ist.

7. Verfahren nach Anspruch 1, dadurch gekennzeichnet, dass es sich um eine Verbindung der Formel I handelt, ausgewählt aus der Reihe:

5-Chlor-2-[(4-chlor-phenyl-sulfonyl)-amino]-benzoesäure-[4-(4-trifluormethyl-6-cyclopropyl-pyrimidin-2-yl-oxy)-anilid];

5-Chlor-2-[(4-chlor-phenyl-sulfonyl)-amino]-benzoesäure-[4-(3-chlor-5-trifluormethyl-pyridin-2-yl-oxy)-anilid];

5-Chlor-2-[(4-chlor-phenyl-sulfonyl)-amino]-benzoesäure-[4-(4-trifluormethyl-pyridin-2-yl-oxy)-anilid];

5-Chlor-2-[(4-chlor-phenyl-sulfonyl)-amino]-benzoesäure-[4-(4-trifluormethyl-6-tert.butyl-pyrimidin-2-yl-oxy)-anilid];

3,5-Dichlor-2-[(4-chlor-phenyl-sulfonyl)-amino]-benzoesäure-[4-(4-trifluormethyl-6-cyclopropyl-pyrimidin-2-yl-oxy)-anilid];

3,5-Dichlor-2-[(4-chlor-phenyl-sulfonyl)-amino]-benzoesäure-[4-(5-trifluormethyl-pyridin-2-yl-oxy)-anilid].

8. Verfahren nach Anspruch 1, dadurch gekennzeichnet, dass es sich um die Verbindung 4-Chlor-2-[(4-chlor-phenyl-sulfonyl)-amino]-benzoesäure-[4-(3-chlor-5-trifluormethyl-pyridin-2-yl-oxy)-anilid] handelt.

9. Verfahren nach einem der Ansprüche 1 bis 8, dadurch gekennzeichnet, dass man bei -20° bis +150° C arbeitet.

10. Verfahren nach Anspruch 1, dadurch gekennzeichnet, dass man bei 0° bis +100° C arbeitet.

11. Verfahren zur Herstellung einer pharmazeutischen Komposition gegen Helminthen beim Warmblüter, dadurch gekennzeichnet, dass man eine Verbindung der Formel I nach einem der Ansprüche 1 bis 8 unter Einschluss ihrer physiologisch verträglichen Säureadditionssalze mit flüssigen und/oder festen Formulierungshilfsstoffen durch schrittweises Vermischen und/oder Vermahlen derart in Kontakt gebracht wird, dass eine anwendungskonforme optimale Entfaltung der anthelmintischen Aktivität der Formulierung erzielt wird.